# NOUVEAU FASCICULE DE BREVET EUROPEEN

(11) **EP 0 904 775 B2**
(45) Date de publication et mention de la décision concernant l'opposition: **16.06.2004**
(45) Mention de la délivrance du brevet: 31.05.2000
(21) Numéro de dépôt: 98401700.4
(22) Date de dépôt: 06.07.1998
(51) Int. Cl.: A61K 7/48, A61K 7/06

(54) **Emulsion contenant de l'acide ascorbique et ses utilisations dans les domaines cosmétique et dermatologique**
Emulsion, die Ascorbinsaüre enthält, und ihre Verwendungen in der Kosmetik und Dermatologie
Emulsion containing ascorbic acid and uses in cosmetic and dermatology

(30) Priorité: 02.09.1997 FR 9710902
(43) Date de publication de la demande: 31.03.1999
(73) Titulaire: L'OREAL, 75008 Paris (FR)
(72) Inventeur: Afriat, Isabelle, 75014 Paris (FR); Chanvin, Florence, 91450 Soisy/S/Seine (FR)
(74) Mandataire: Dossmann, Gérard

(56) Documents cités:
- EP-A- 0 670 157
- EP-A- 0 755 674
- WO-A-94/09756
- WO-A-98/00102
- FR-A- 2 738 743

## Description

L'invention se rapporte à une émulsion eau-dans-huile (E/H) contenant de l'acide ascorbique, à l'utilisation de cette émulsion pour le traitement de la peau par voie topique, sur le visage, y compris autour des yeux, sur le corps ainsi que sur le cuir chevelu des êtres humains, ainsi qu'à un procédé de traitement topique de la peau à l'aide de cette émulsion.

On cherche depuis longtemps à stabiliser l'acide ascorbique ou vitamine C, dans des présentations galéniques appropriées, du fait de ses propriétés bénéfiques.

En effet, l'acide ascorbique possède de nombreuses fonctions biologiques comme la stimulation de la synthèse du collagène, le renfort des tissus cutanés contre les agressions extérieures, la dépigmentation, l'activité anti-radicaux libres, la compensation de la déficience en vitamine E.

Cependant, en raison de sa structure chimique (d'alpha-cétolactone), l'acide ascorbique est très sensible à l'influence des paramètres de l'environnement comme la lumière, l'oxygène et l'eau. Il s'ensuit une dégradation inéluctable au cours du temps de l'acide ascorbique en solution, entraînant une perte de son activité. Par ailleurs, la dégradation même faible de l'acide ascorbique provoque un jaunissement de la composition le contenant.

Pour diminuer ou retarder la dégradation de l'acide ascorbique en solution, il a été préconisé dans le document US-A-5,140,043, de l'introduire dans des solutions hydroalcooliques formées d'au moins 80 % d'eau et ayant un pH inférieur à 3,5. Toutefois, une application répétée de solutions à pH fortement acide sur la peau peut perturber l 'équilibre de la peau et en particulier provoquer des irritations de la peau.

Par ailleurs, le document EP-A-670157 décrit la stabilisation de la vitamine C dans une émulsion E/H ayant un pH au plus égal à 3,5 et contenant comme agent émulsionnant, un diméthiconecopolyol et/ou un alkyldiméthiconecopolyol. Une telle émulsion E/H est mieux supportée par les utilisateurs qu'une solution hydroalcoolique, du fait que la phase aqueuse acide contenant l'acide ascorbique est appliquée en faible quantité sur la peau, à l'état de fines gouttelettes dispersées dans l'huile, ce qui ne provoque pas d'irritation ni de brûlure de la peau. Toutefois, l'application répétée d'une composition à un pH acide peut avoir des inconvénients chez les sujets à peaux sensibles.

En outre, dans le document WO-A-95/28092, il a été préconisé de stabiliser les actifs tels qu'enzymes ou vitamines, dans une émulsion eau-dans-huile comportant une concentration élevée de polyols et un émulsifiant choisi parmi les monoglycérides et le polyricinoléate de glycérol. Toutefois, les émulsions décrites dans ce document comportent moins de 10 % d'eau et il subsiste le besoin d'émulsions pouvant comporter plus d'eau tout en maintenant une bonne stabilité de l'acide ascorbique.

Il subsiste donc le besoin d'une composition utilisable dans les domaines cosmétique et/ou dermatologique, dans laquelle l'acide ascorbique soit stable et qui ne provoque aucune irritation de la peau après application, même chez les sujets à peaux sensibles.

La demanderesse a maintenant trouvé de manière surprenante qu'en ajustant à un pH de 5,5 à 7,5, la phase aqueuse d'une émulsion eau-dans-huile comportant un tensioactif siliconé, telle que décrite dans le document EP-A-670157, on améliorait notablement la stabilité de l'acide ascorbique tout en obtenant une composition très bien tolérée par la peau humaine.

La présente invention a donc pour objet une émulsion contenant de l'acide ascorbique et comprenant une phase aqueuse dispersée dans une phase huileuse à l'aide d'au moins un agent émulsionnant siliconé, caractérisée en ce que la phase aqueuse présente un pH allant de 5,5 à 7,5, de préférence égal à 6 à l'exception des emulsions comprenant un système redox t-butylhydroxypheroxyde/acide ascorbique.

Certes il est connu que le pH a une influence sur la stabilité de l'acide ascorbique. Ainsi, l'article de B.R. Hajratwala intitulé "Stability of ascorbic acid", paru dans la Revue Sciences Pharmaceutiques, le 15 Mars 1985, montre que la décomposition de l'acide ascorbique présente des minima à pH 2,5-3 et à pH6. Toutefois, tous les essais présentés dans cet article sont réalisés à partir de solutions simples d'acide ascorbique dans l'eau et l'homme du métier ne peut en déduire le comportement de l'acide ascorbique dans une émulsion où les interactions des huiles et des tensioactifs modifient notablement et de manière imprévisible l'environnement.

Ainsi, la demanderesse a trouvé de manière surprenante que, contrairement à l'enseignement de cet article selon lequel l 'acide ascorbique en solution a un comportement très différent en milieu aérobie et en milieu anaérobie, la dégradation de l'acide ascorbique dans une émulsion selon l'invention, est la même, que le milieu soit aérobie ou anaérobie.

En outre, les documents de l'art antérieur dans le domaine cosmétique incitent l'homme du métier à utiliser un pH acide (voir US-A-5,140,043 et EP-A-670157 cités ci-dessus).

Dans l'émulsion selon l'invention, le pH est de préférence égal ou proche de 6. Ce pH est proche de celui de la peau ; il s'ensuit une grande compatibilité de l'émulsion de l'invention vis-à-vis de la peau.

L'émulsion selon l'invention permet d'éviter la dégradation de l'acide ascorbique et donc de le stabiliser. Aussi, la présente invention a encore pour objet à l'utilisation de l'émulsion telle que définie ci-dessus pour stabiliser l'acide ascorbique.

Les concentrations en acide ascorbique dans l'émulsion de l'invention sont celles classiquement utilisées dans les domaines cosmétique et dermatologique et, par exemple, de 0,01 à 20 %, de préférence de 0,1 à 10 % et mieux de 0,5 à % du poids total de l'émulsion.

L'agent émulsionnant siliconé est de préférence choisi parmi les diméthiconecopolyols et les alkyldiméthiconecopolyols. Ces émulsionnants comprennent de préférence des groupements polyéthers totalement oxyéthylénés. Toutefois, il est possible d'utiliser aussi des émulsionnants ayant des groupements polyéthers partiellement oxyéthylénés.

De manière avantageuse, le pourcentage en poids de polyéther par rapport au poids total de l'agent émulsionnant est choisi de 1 à 50 %, de préférence de 15 à 35 % dans le cas des diméthiconecopolyols, et de 1 à 5 %, de préférence de 2 à 3 % dans le cas des alkyldiméthiconecopolyols.

Comme diméthiconecopolyol utilisable dans l'émulsion selon l'invention, on peut citer le mélange de diméthiconecopolyol et de cyclométhicone vendu sous la dénomination "Q2-3225C" par la Société DOW CORNING et le produit vendu sous la dénomination "SF-1228" par la Société GENERAL ELECTRIC.

Comme alkyldiméthiconecopolyol utilisable dans l'émulsion selon l'invention, on peut utiliser le lauryldiméthiconecopolyol tel que, par exemple, celui vendu sous la dénomination "Q2-5200" par la Société DOW CORNING, et le cétyldiméthiconecopolyol tel que, par exemple, celui vendu sous la dénomination "ABIL EM 90" par la Société GOLDSCHMIDT

Les agents émulsionnants utilisés dans l'émulsion de l'invention peuvent être associés en outre à au moins un coémulsionnant tel que, notamment, le tétraisostéarate de polyglycérol ou le trioléate de polyglycérol.

Les agents émulsionnants sont, par exemple, présents dans l'émulsion selon l'invention en une concentration allant de 0,5 à 25 %, et de préférence de 5 à 20 % lorsqu'ils sont employés seuls. Lorsque la teneur en agent émulsionnant est inférieure à 2,5 % du poids total de l'émulsion, il est préférable d'ajouter un coémulsionnant. Quand il est présent, le coémulsionnant est utilisé à raison de 1 fois à 10 fois la quantité pondérale de l'agent émulsionnant. De manière avantageuse, les concentrations respectives d'agents émulsionnants et coémulsionnants vont de 0,5 à 10 % et de 3 à 7 % du poids total de l'émulsion.

Pour des températures supérieures à 20°C et/ou pour des périodes de stockage de plusieurs mois, on préfère utiliser comme agent émulsionnant un alkyldiméthiconecopolyol totalement oxyéthyléné et plus spécialement, le diméthiconecopolyol.

Pour ajuster le pH de l'émulsion, on peut utiliser tout agent basique approprié, et notamment les bases minérales comme les hydroxydes de métaux alcalins (hydroxydes de sodium et de potassium) ou les hydroxydes d'ammonium, et les bases organiques, notamment les bases amphotères, c'est-à-dire des bases ayant à la fois des groupements fonctionnels anioniques et cationiques.

Les bases amphotères peuvent être des amines organiques primaires, secondaires, tertiaires ou cycliques et plus spécialement des acides aminés. A titre d'exemple de bases amphotères, on peut citer la glycine, la lysine, l'arginine, la taurine, l'histidine, l'alanine, la valine, la cystéïne, la trihydroxyméthylaminométhane (TRISTA), la triéthanolamine.

On peut utiliser un ou plusieurs agents basiques. La quantité d'agent(s) basique(s) doit être suffisante pour amener le pH de l'émulsion entre 5,5 et 7,5 et de préférence à pH 6. Cette quantité peut aller par exemple de 0,1 à 10 % et de préférence de 0,5 à 3 % du poids total de l'émulsion.

Au lieu d'ajouter un agent basique, on peut incorporer dans l'émulsion l'acide ascorbique directement sous forme de sel, et par exemple sous forme d'un sel de métal alcalin, d'ammonium ou de base organique. Comme sel, on peut citer par exemple l'ascorbate de sodium ou l'ascorbate de calcium.

Par ailleurs, on peut ajouter à l'agent basique une faible quantité d'un acide permettant d'obtenir un effet tampon, tel que l'acide citrique. Cet acide peut être utilisé par exemple en une quantité allant de 0,1 à 5 % du poids total de l'émulsion.

Selon l'invention, la phase aqueuse de l'émulsion peut représenter de 25 à 90 % et, de préférence, de 45 à 80 % du poids total de l'émulsion.

La phase aqueuse de l'émulsion peut, en outre, contenir un électrolyte, comme par exemple le chlorure de sodium ou de potassium, de manière à améliorer encore la stabilité de l'émulsion. La teneur en électrolyte dans l'émulsion peut aller de 0 à 3 % et de préférence de 0,5 à 2 % du poids total de l'émulsion.

De façon avantageuse, et afin d'éviter notamment la présence dans la phase aqueuse de métaux lourds pouvant catalyser la dégradation de l'acide ascorbique, la phase aqueuse est formée d'eau permutée ou désionisée.

Pour augmenter encore la stabilité de l'acide ascorbique au cours du temps, l'émulsion de l'invention peut comprendre un agent séquestrant les métaux tel qu'un dérivé d'acide phosphonique.

Les dérivés d'acide phosphonique utilisables dans l'invention sont notamment choisis parmi l'acide éthylènediamine tétra(méthylène phosphonique), l'acide hexaméthylènediamine tétra(méthylène phosphonique), l'acide diéthylènetriamine penta(méthylènephosphonique), et leurs sels et notamment leur sels de sodium comme le sel pentasodique de l'acide éthylènediamine tétra(méthylène phosphonique).

De manière avantageuse, on utilise l'acide éthylènediamine tétra(méthylène phosphonique), notamment celui vendu par la Société MONSANTO sous la dénomination Dequest 2041. On peut aussi utiliser avantageusement le sel pentasodique de ce dernier qui est vendu sous la dénomination Dequest 2046 par la Société MONSANTO. Comme autre agent séquestrant utilisable dans l'émulsion de l'invention, on peut citer l'acide diéthylène triamine pentaacétique, vendu par exemple par la Société SIGMA.

Lorsqu'il est présent, l'agent séquestrant est en une concentration allant en général de 0,005 à 0,2 % du poids total de l'émulsion.

Selon l'invention, la phase huileuse de l'émulsion peut représenter de 3 à 75 % et, de préférence, de 5 à 30 % du poids total de l'émulsion. La phase huileuse de l'émulsion de l'invention peut renfermer toute sorte d'huiles et de corps gras bien connus de l'homme du métier, comme les huiles minérales (paraffine, vaseline), les huiles d'origine végétale (huile d'amande, huile d'abricot, huile de jojoba), les huiles synthétiques (perhydrosqualène, polyisobutène hydrogéné), les huiles de silicone volatiles ou non.

De préférence, la phase huileuse de l'émulsion de l'invention comprend au moins une huile de silicone volatile généralement à raison de 3 à 15 % du poids total de l'émulsion, comme, par exemple, une huile de silicone cyclique telle que la cyclopentadiméthylsiloxane ou la cyclohexadiméthylsiloxane. Elle peut aussi comprendre une huile de silicone non volatile comme, par exemple, la phényltriméthicone telle que "Dow Corning 556 Fluid" vendu par DOW CORNING.

Selon un mode particulier de réalisation de l'invention, la phase huileuse de l'émulsion est entièrement constituée d'huile de silicone.

La phase huileuse peut comprendre aussi d'autres corps gras que les huiles, choisis parmi les alcools gras, les acides gras, les cires, les résines (par exemple résine de silicone).

La phase huileuse peut comprendre, en outre, un agent gélifiant. Comme agent gélifiant, on peut citer les argiles modifiées comme les bentones, les sels métalliques d'acides gras comme les stéarates d'aluminium et la silice hydrophobe. L'agent gélifiant de la phase huileuse peut être présent en une concentration allant, par exemple, de 1 à 15 %, de préférence 3 à 7 % du poids total de l'émulsion.

Selon un mode particulier de réalisation de l'invention, l'émulsion contient des polyols en une quantité suffisante pour améliorer encore la stabilité de l'acide ascorbique. Les polyols peuvent, par exemple, être choisis parmi la glycérine, les glycols tels que le propylène glycol et le PEG 8, et les silicones comportant des groupes hydroxyle. Les polyols sont présents en une quantité allant, de préférence, de 0,5 à 30 % et préférentiellement de 10 à 25 % du poids total de l'émulsion.

De façon connue, l'émulsion de l'invention peut contenir également des additifs habituels dans les domaines cosmétique et dermatologique, tels que les actifs hydrophiles ou lipophiles autres que l'acide ascorbique, les conservateurs, les antioxydants, les solvants, les parfums, les charges, les filtres, les absorbeurs d'odeur et les matières colorantes, dans la mesure où l'additif ne déstabilise pas l'acide ascorbique dans l'émulsion. Les quantités de ces différents additifs sont celles classiquement utilisées dans les domaines considérés, et par exemple de 0,01 à 15 % du poids total de la composition. Ces additifs, selon leur nature, peuvent être introduits dans la phase grasse ou dans la phase aqueuse.

Comme charge, on peut citer par exemple le nylon, l'amidon et ses dérivés.

Comme actifs hydrophiles, on peut utiliser par exemple, outre les polyols indiqués ci-dessus, les protéines ou les hydrolysats de protéine, le pyrrolidone carboxylate de sodium, les NMF (facteurs normaux d'hydratation), l'acide hyaluronique, les acides aminés, l'allantoïne, les sucres et les dérivés de sucre, l'amidon.

Comme actifs lipophiles, on peut utiliser par exemple le tocophérol (vitamine E) et ses dérivés, les acides gras essentiels, les céramides, les huiles essentielles.

De façon avantageuse, la composition de l'invention comprend, en poids :
- de 0,01 à 20 % d'acide ascorbique,
- de 0,5 à 25 % de diméthiconecopolyol,
- de 0,1 à 10 % d'huile de silicone,
- de 0,005 à 0,2 % de sel pentasodique de l'acide éthylène tétra(méthylène phosphonique), et
- de 0,1 à 10 % d'hydroxyde de sodium.

Comme déjà mentionné ci-dessus, la dégradation de l'acide ascorbique si minime soit elle, entraîne un jaunissement de la composition le contenant. C'est pourquoi il est préférable, pour éviter ce jaunissement, que l'émulsion selon l'invention soit conditionnée de sorte à ne pas être en contact avec l'oxygène et à être à l'abri de la lumière.

Aussi, l'émulsion de l'invention est, de préférence, préparée sous atmosphère inerte (azote ou gaz rare tel que l'argon ), exempte de tout oxygène ou contenant moins de 3% v/v d'oxygène, et sous lumière inactinique, telle que celle d'une lampe à vapeur de sodium.

De manière avantageuse, l'émulsion de l'invention est conditionnée en présence d'un absorbeur d'oxygène tel que, par exemple, l'absorbeur d'oxygène "Atco" vendu par la Société STANDA INDUSTRIES. L'absorbeur d'oxygène est de préférence séparé de l'émulsion par une membrane poreuse aux gaz et imperméable aux liquides, telle que celle décrite dans le document FR-A-2671055.

De manière encore plus préférée, l'émulsion de l'invention est conditionnée dans un conteneur surmonté d'un dispositif de distribution sans reprise d'air comme, par exemple, celui décrit dans le document FR-A-2666308.

L'invention a aussi pour objet une composition cosmétique et/ou dermatologique comprenant une émulsion telle que définie précédemment.

Pour une application cosmétique et/ou dermatologique, l'émulsion selon l'invention doit contenir un milieu topiquement acceptable, c'est-à-dire compatible avec la peau, les muqueuses et/ou les cheveux. La composition à base de cette émulsion peut constituer notamment des compositions de nettoyage, de protection, de traitement ou de soin de la peau et/ou des cheveux, en particulier pour le visage, pour le cou, pour les mains, pour les cheveux, pour le cuir chevelu ou pour le corps, ainsi que pour les cils.

L'invention a encore pour objet l'utilisation de la composition cosmétique selon l'invention ci-dessus pour le traitement cosmétique de la peau, et en particulier pour lisser les ridules de la peau, la tonifier, la régénérer, pour éclaircir le teint, éliminer les taches pigmentaires de la peau, pour lutter contre les méfaits des rayonnements UV, et/ou pour renforcer de manière générale les tissus cutanés contre les agressions de l'environnement (pollution).

L'invention a également pour objet l'utilisation de l'émulsion ci-dessus pour la fabrication d'une crème destinée à un traitement dermatologique de la peau.

L'invention a enfin pour objet un procédé de traitement cosmétique de la peau, consistant à appliquer sur la peau, y compris autour des yeux, une composition cosmétique conforme à l'invention.

Les exemples ci-après de compositions selon l'invention, sont donnés à titre d'illustration et sans caractère limitatif. Les quantités y sont données en % en poids, sauf mention contraire.

### Exemple 1 : Crème pour le visage

- Mélange de diméthiconecopolyol et de cyclométhicone (Q2-3225C de Dow Corning) 20 %
- Phényltriméthicone (Dow Corning 556 Fluid) 4 %
- Huile végétale 3 %
- Glycérine 23 %
- Propylène glycol 6 %
- Hydroxyde de sodium 1,83 %
- Acide citrique 1,24 %
- Acide ascorbique 5 %
- Eau permutée qsp 100%

L'émulsion obtenue présente un pH de 6. Elle se présente sous forme d'une crème appropriée pour le soin du visage, douce à l'application. Cette crème procure un éclat du teint immédiat et permet un lissage des imperfections.

Par ailleurs, l'acide ascorbique dans cette émulsion présente une dégradation de 8 % après deux mois à 45°C.

### Exemple 1 comparatif :

On a réalisé un exemple comparatif en remplaçant dans l'exemple 1, l'acide citrique et la soude par de l'eau. L'émulsion obtenue a alors un pH de 2,8. Cette émulsion présente une dégradation de 17 % après deux mois à 45°C ; cette dégradation est donc beaucoup plus importante que celle de l'émulsion à pH 6.

### Exemple 2 Crème pour le visage

- Mélange de diméthiconecopolyol et de cyclométhicone (Q2-3225C de Dow Corning) 8 %
- Phényltriméthicone (Dow Corning 556 Fluid) 15%
- Tocophérol 0,5 %
- Propylène glycol 10 %
- PEG-8 8%
- Glycérine 2,4 %
- Sel pentasodique de l'acide éthylène tétra(méthylène phosphonique) dans l'eau à 33 % 0,1 %
- Hydroxyde de sodium 1,74 %
- Acide citrique 1,24 %
- Acide ascorbique 5 %
- Eau permutée qsp 100 %

Cette crème pour le soin du visage est légère et douce. Elle procure un éclat du teint immédiat et permet un lissage des imperfections.

Par ailleurs, l'acide ascorbique qu'elle contient a subi une dégradation de 8 % après deux mois à 45°C.

## Revendications

1. Emulsion contenant de l'acide ascorbique et comprenant une phase aqueuse dispersée dans une phase huileuse l'aide d'au moins un agent émulsionnant siliconé, **caractérisée en ce que** la phase aqueuse présente un pH allant de 5,5 à 7,5, à l'exception des émulsions comprenant un système redox t-butylhydroxyperoxyde/acide ascorbique

2. Emulsion selon la revendication 1, **caractérisée en ce que** la phase aqueuse présente un pH de 6.

3. Emulsion selon la revendication 1 ou 2, **caractérisée en ce que** l'acide ascorbique est présent à une concentration allant de 0,1 à 10 % du poids total de l'émulsion.

4. Emulsion selon l'une quelconque des revendications précédentes, **caractérisée en ce que** la valeur du pH de la phase aqueuse est ajustée à l'aide d'au moins un agent basique choisi parmi les bases minérales et les bases organiques.

5. Emulsion selon la revendication précédente, **caractérisée en ce que** l'agent basique est présent en une quantité allant de 0,1 à 10% du poids total de l'émulsion.

6. Emulsion selon l'une quelconque des revendications précédentes, **caractérisée en ce que** l'acide ascorbique est incorporé sous forme de sel.

7. Emulsion selon l'une quelconque des revendications précédentes, **caractérisée en ce qu'**elle contient, en outre, de l'acide citrique.

8. Emulsion selon l'une quelconque des revendications précédentes, **caractérisée en ce que** la phase aqueuse représente de 25 à 90 % du poids total de l'émulsion.

9. Emulsion selon l'une quelconque des revendications précédentes, **caractérisée en ce que** l'agent émulsionnant est choisi parmi les diméthiconecopolyols et les alkyldiméthiconecopolyols.

10. Emulsion selon l'une quelconque des revendications précédentes, **caractérisée en ce que** l'agent émulsionnant comporte des groupements polyéthers totalement oxyéthylénés.

11. Emulsion selon l'une quelconque des revendications précédentes, **caractérisée en ce qu'**elle contient, en outre, un coémulsionnant utilisé en une concentration allant de 1 fois à 10 fois la quantité pondérale de l'agent émulsionnant.

12. Emulsion selon l'une quelconque des revendications précédentes, **caractérisée en ce que** l'agent émulsionnant est présent à une concentration allant de 0,5 à 25 % du poids total de l'émulsion.

13. Emulsion selon l'une quelconque des revendications précédentes, **caractérisée en qu**'elle comprend, en outre, un agent séquestrant les métaux.

14. Emulsion selon la revendication précédente, **caractérisée en ce que** l'agent séquestrant est le sel pentasodique de l'acide éthylénediamine tétra(méthylène phosphonique).

15. Composition cosmétique et/ou dermatologique, **caractérisée en ce qu'**elle comprend une émulsion selon l'une quelconque des revendications précédentes.

16. Utilisation d'une composition cosmétique selon la revendication précédente pour un traitement cosmétique de la peau en vue de la tonifier, de la régénérer, de lisser les ridules de la peau, d'éclaircir le teint, d'éliminer les taches pigmentaires de la peau, et/ou pour lutter contre les méfaits des rayonnements UV, et/ou pour renforcer les tissus cutanés contre les agressions de l'environnement.

17. Utilisation d'une émulsion selon l'une quelconque des revendications 1 à 14 pour la fabrication d'une crème destinée à un traitement dermatologique de la peau.

18. Procédé de traitement cosmétique de la peau, **caractérisé en ce qu'**il consiste à appliquer sur la peau, y compris autour des yeux, une composition selon la revendication 15.

19. Utilisation de l'émulsion selon l'une quelconque des revendications 1 à 14 pour stabiliser l'acide ascorbique.

## Patentansprüche

1. Emulsion, die Ascorbinsäure enthält und eine wässerige Phase aufweist, die mit mindestens einem siliconhaltigen Emulgator in einer Ölphase dispergiert ist, **dadurch gekennzeichnet, dass** die wässerige Phase einen pH-Wert im Bereich von 5,5 bis 7,5 aufweist, wobei Emulsionen ausgenommen sind, die ein *t*-Butylhydroperoxid/Ascorbinsäure-Redoxsystem enthalten.

2. Emulsion nach Anspruch 1, **dadurch gekennzeichnet, dass** die wässerige Phase einen pH-Wert von 6 aufweist.

3. Emulsion nach Anspruch 1 oder 2, **dadurch gekennzeichnet, dass** die Ascorbinsäure in einer Konzentration im Bereich von 0,1 bis 10 % des Gesamtgewichts der Emulsion vorliegt.

4. Emulsion nach einem der vorhergehenden Ansprüche, **dadurch gekennzeichnet, dass** der pH-Wert der wässerigen Phase mit mindestens einem basischen Mittel eingestellt wird, das unter anorganischen und organischen Basen ausgewählt ist.

5. Emulsion nach dem vorhergehenden Anspruch, **dadurch gekennzeichnet, dass** das basische Mittel in einem Mengenanteil von 0,1 bis 10 % des Gesamtgewichts der Emulsion vorliegt.

6. Emulsion nach einem der vorhergehenden Ansprüche, **dadurch gekennzeichnet, dass** die Ascorbinsäure in Form eines Salzes eingebracht wird.

7. Emulsion nach einem der vorhergehenden Ansprüche, **dadurch gekennzeichnet, dass** sie ferner Citronensäure enthält.

8. Emulsion nach einem der vorhergehenden Ansprüche, **dadurch gekennzeichnet, dass** die wässerige Phase 25 bis 90 % des Gesamtgewichts der Emulsion ausmacht.

9. Emulsion nach einem der vorhergehenden Ansprüche, **dadurch gekennzeichnet, dass** der Emulgator unter den Dimeticoncopolyolen und den Alkyldimeticoncopolyolen ausgewählt ist.

10. Emulsion nach einem der vorhergehenden Ansprüche, **dadurch gekennzeichnet, dass** der Emulgator vollständig ethoxylierte Polyethergruppen aufweist.

11. Emulsion nach einem der vorhergehenden Ansprüche, **dadurch gekennzeichnet, dass** sie ferner einen Coemulgator enthält, der in einer Konzentration verwendet wird, die das einfache bis zehnfache der Gewichtsmenge des Emulgators beträgt.

12. Emulsion nach einem der vorhergehenden Ansprüche, **dadurch gekennzeichnet, dass** der Emulgator in einer Konzentration im Bereich von 0,5 bis 25 % des Gesamtgewichts der Emulsion vorliegt.

13. Emulsion nach einem der vorhergehenden Ansprüche, **dadurch gekennzeichnet, dass** sie ferner ein Maskierungsmittel für Metalle enthält.

14. Emulsion nach dem vorhergehenden Anspruch, **dadurch gekennzeichnet, dass** das Maskierungsmittel das Pentanatriumsalz von Ethylendiamintetra-(methylenphosphonsäure) ist.

15. Kosmetische und/oder dermatologische Zusammensetzung, **dadurch gekennzeichnet, dass** sie eine Emulsion nach einem der vorhergehenden Ansprüche enthält.

16. Verwendung einer kosmetischen Zusammensetzung nach dem vorhergehenden Anspruch zur kosmetischen Behandlung der Haut, um die Haut zu beleben und zu regenerieren, die Hautfältchen zu glätten, den Teint aufzufrischen, Pigmentflecken der Haut zu beseitigen und/ oder die schädlichen Folgen der UV-Bestrahlung zu bekämpfen und/oder die Hautgewebe gegenüber Umwelteinwirkungen zu schützen.

17. Verwendung einer Emulsion nach einem der.Ansprüche 1 bis 14 zur Herstellung einer Creme, die zur dermatologischen Behandlung der Haut bestimmt ist.

18. Verfahren zur kosmetischen Behandlung der Haut, **dadurch gekennzeichnet, dass** es darin besteht, eine Zusammensetzung nach Anspruch 15 auf die Haut einschließlich der Augenpartie aufzutragen.

19. Verwendung der Emulsion nach einem der Ansprüche 1 bis 14 zur Stabilisierung von Ascorbinsäure.

## Claims

1. Emulsion containing ascorbic acid and comprising an aqueous phase dispersed in an oily phase using at least one silicone-based emulsifier, **characterized in that** the aqueous phase has a pH ranging from 5.5 to 7.5, with the exception of emulsions comprising a t-butyl hydroxyperoxide/ascorbic acid redox system.

2. Emulsion according to Claim 1, **characterized in that** the aqueous phase has a pH of 6.

3. Emulsion according to Claim 1 or 2, **characterized in that** the ascorbic acid is present in a concentration ranging from 0.1 to 10% of the total weight of the emulsion.

4. Emulsion according to any one of the preceding claims, **characterized in that** the value of the pH of the aqueous phase is adjusted using at least one basic agent chosen from inorganic bases and organic bases.

5. Emulsion according to the preceding claim, **characterized in that** the basic agent is present in an amount ranging from 0.1 to 10% of the total weight of the emulsion.

6. Emulsion according to any one of the preceding claims, **characterized in that** the ascorbic acid is incorporated in salt form.

7. Emulsion according to any one of the preceding claims, **characterized in that** it also contains citric acid.

8. Emulsion according to any one of the preceding claims, **characterized in that** the aqueous phase represents from 25 to 90% of the total weight of the emulsion.

9. Emulsion according to any one of the preceding claims, **characterized in that** the emulsifier is chosen from dimethiconecopolyols and alkyldimethiconecopolyols.

10. Emulsion according to any one of the preceding claims, **characterized in that** the emulsifier contains fully oxyethylenated polyether groups.

11. Emulsion according to any one of the preceding claims, **characterized in that** it also contains a co-emulsifier used in a concentration ranging from once to 10 times the weight amount of the emulsifier.

12. Emulsion according to any one of the preceding claims, **characterized in that** the emulsifier is present in a concentration ranging from 0.5 to 25% of the total weight of the emulsion.

13. Emulsion according to any one of the preceding claims, **characterized in that** it also comprises a metal-sequestering agent.

14. Emulsion according to the preceding claim, **characterized in that** the sequestering agent is the pentasodium salt of ethylenediaminetetra-(methylenephosphonic acid).

15. Cosmetic and/or dermatological composition, **characterized in that** it comprises an emulsion according to any one of the preceding claims.

16. Use of a cosmetic composition according to the preceding claim, for a cosmetic treatment of the skin in order to tonify and regenerate it, to smooth out fine lines on the skin, to improve the complexion, to remove skin pigmentation marks and/or to combat the harmful effects of UV radiation, and/or to strengthen skin tissues against environmental attack.

17. Use of an emulsion according to any one of Claims 1 to 14, for the manufacture of a cream intended for a dermatological treatment of the skin.

18. Cosmetic process for treating the skin, **characterized in that** it consists in applying a composition according to Claim 15 to the skin, including the area around the eyes.

19. Use of the emulsion according to any one of Claims 1 to 14, to stabilize ascorbic acid.
